# EUROPEAN PATENT APPLICATION

(11) **EP 1 978 360 A1**
(43) Date of publication of application: **08.10.2008**
(21) Application number: 07105682.4
(22) Date of filing: 04.04.2007
(51) Int. Cl.: G01N 33/50, C12N 5/06

(54) **A method to improve barrier function of cell-cell junctions**

(71) Applicant: UMC Utrecht Holding B.V., 3584 CM Utrecht (NL)
(72) Inventor: Bos, Johannes Lucas, 3981 BG Bunnik (NL); Dubé, Nadia Marie-Noël, 3512 CV Utrecht (NL); Kooistra, Matthéüs Ritske Harm, 3532 EA Utrecht (NL)
(74) Representative: van Loon, C.J.J.

(57) **Abstract**

The present invention discloses a method to increase the barrier function of a cell-cell junction, said method comprising the activation of a PDZ-GEF2 protein, wherein said PDZ-GEF2 protein comprises an amino acid sequence encoded by a gene corresponding to the RapGEF6 gene. The invention further discloses a method for selecting an activator of PDZ-GEF2, said method comprising contacting a plurality of candidate compounds to at least two cells of a first cell type and at least two cells of a second cell type, both cell types capable of forming cell-cell junctions, measuring the maturation of cell-cell junctions before and after contacting said two cell types with said plurality of candidate compounds, and selecting a compound of said plurality of candidate compounds that is more capable of increasing the integrity of a cell-cell junction between said at least two cells of said first cell type, as compared to its capability of increasing the integrity of a cell-cell junction between cells of said second type,, wherein in said second cell type the amount and/or activity of PDZ-GEF2 is decreased, compared to said first cell type.

SEQUENCE LISTING

<110>  UMC Utrecht Holding B.V.

<120>  A new method to improve barrier function of cell-cell junctions

<130>  P79603EP00

<140>  EP 07105682.4
<141>  2007-04-04

<160>  7

<170>  PatentIn version 3.3

<210>  1
<211>  52
<212>  DNA
<213>  Artificial

<220>
<223>  Fd primer

<400>  1
ggggacaagt ttgtacaaaa aagcaggctc tagagatgag aatctggaac cg          52

<210>  2
<211>  56
<212>  DNA
<213>  Artificial

<220>
<223>  Rv primer

<400>  2
ggggaccact ttgtacaaga aagctgggtc ttaacggcat aaagtaaggt tgacat      56

<210>  3
<211>  19
<212>  DNA
<213>  Artificial

<220>
<223>  PDZ-GEF2 285

<400>  3
ggatccaact tatatagaa                                               19

<210>  4
<211>  19
<212>  DNA
<213>  Artificial

<220>
<223>  PDZ-GEF2 289

<400>  4
gacacggatt gtattatta                                               19

<210>  5
<211>  19
<212>  DNA
<213>  Artificial

<220>
<223>  *Epac1(1)

<400>  5
gcacctacgt ctgcaacaa                                                    19


<210>  6
<211>  19
<212>  DNA
<213>  Artificial

<220>
<223>  *Epac1(2)

<400>  6
ccatcatcct gcgagaaga                                                    19


<210>  7
<211>  1601
<212>  PRT
<213>  Homo sapiens

<400>  7

Met Asn Ser Pro Val Asp Pro Gly Ala Arg Gln Ala Leu Arg Lys Lys
1               5                   10                  15

Pro Pro Glu Arg Thr Pro Glu Asp Leu Asn Thr Ile Tyr Ser Tyr Leu
            20                  25                  30

His Gly Met Glu Ile Leu Ser Asn Leu Arg Glu His Gln Leu Arg Leu
        35                  40                  45

Met Ser Ala Arg Ala Arg Tyr Glu Arg Tyr Ser Gly Asn Gln Val Leu
    50                  55                  60

Phe Cys Ser Glu Thr Ile Ala Arg Cys Trp Tyr Ile Leu Leu Ser Gly
65                  70                  75                  80

Ser Val Leu Val Lys Gly Ser Met Val Leu Pro Pro Cys Ser Phe Gly
                85                  90                  95

Lys Gln Phe Gly Gly Lys Arg Gly Cys Asp Cys Leu Val Leu Glu Pro
            100                 105                 110

Ser Glu Met Ile Val Val Glu Asn Ala Lys Asp Asn Glu Asp Ser Ile
        115                 120                 125

Leu Gln Arg Glu Ile Pro Ala Arg Gln Ser Arg Arg Arg Phe Arg Lys
    130                 135                 140

Ile Asn Tyr Lys Gly Glu Arg Gln Thr Ile Thr Asp Asp Val Glu Val
145                 150                 155                 160

Asn Ser Tyr Leu Ser Leu Pro Ala Asp Leu Thr Lys Met His Leu Thr
                165                 170                 175

Glu Asn Pro His Pro Gln Val Thr His Val Ser Ser Ser Gln Ser Gly
            180                 185                 190

Cys Ser Ile Ala Ser Asp Ser Gly Ser Ser Ser Leu Ser Asp Ile Tyr
        195                 200                 205

Gln Ala Thr Glu Ser Glu Val Gly Asp Val Asp Leu Thr Arg Leu Pro
    210                 215                 220

Glu Gly Pro Val Asp Ser Glu Asp Asp Glu Glu Glu Asp Glu Glu Ile
225                 230                 235                 240

Asp Arg Thr Asp Pro Leu Gln Gly Arg Asp Leu Val Arg Glu Cys Leu
                245                 250                 255

Glu Lys Glu Pro Ala Asp Lys Thr Asp Asp Asp Ile Glu Gln Leu Leu
            260                 265                 270

Glu Phe Met His Gln Leu Pro Ala Phe Ala Asn Met Thr Met Ser Val
        275                 280                 285

Arg Arg Glu Leu Cys Ser Val Met Ile Phe Glu Val Val Glu Gln Ala
    290                 295                 300

Gly Ala Ile Ile Leu Glu Asp Gly Gln Glu Leu Asp Ser Trp Tyr Val
305                 310                 315                 320

Ile Leu Asn Gly Thr Val Glu Ile Ser His Pro Asp Gly Lys Val Glu
                325                 330                 335

Asn Leu Phe Met Gly Asn Ser Phe Gly Ile Thr Pro Thr Leu Asp Lys
            340                 345                 350

Gln Tyr Met His Gly Ile Val Arg Thr Lys Val Asp Asp Cys Gln Phe
        355                 360                 365

Val Cys Ile Ala Gln Gln Asp Tyr Trp Arg Ile Leu Asn His Val Glu
    370                 375                 380

Lys Asn Thr His Lys Val Glu Glu Glu Gly Glu Ile Val Met Val His
385                 390                 395                 400

Glu His Arg Glu Leu Asp Arg Ser Gly Thr Arg Lys Gly His Ile Val
                405                 410                 415

Ile Lys Ala Thr Pro Glu Arg Leu Ile Met His Leu Ile Glu Glu His
            420                 425                 430

Ser Ile Val Asp Pro Thr Tyr Ile Glu Asp Phe Leu Leu Thr Tyr Arg
        435                 440                 445

Thr Phe Leu Glu Ser Pro Leu Asp Val Gly Ile Lys Leu Leu Glu Trp
    450                 455                 460

Phe Lys Ile Asp Ser Leu Arg Asp Lys Val Thr Arg Ile Val Leu Leu
465                 470                 475                 480

Trp Val Asn Asn His Phe Asn Asp Phe Glu Gly Asp Pro Ala Met Thr
                485                 490                 495

Arg Phe Leu Glu Glu Phe Glu Lys Asn Leu Glu Asp Thr Lys Met Asn
            500                 505                 510

Gly His Leu Arg Leu Leu Asn Ile Ala Cys Ala Ala Lys Ala Lys Trp
        515                 520                 525

Arg Gln Val Val Leu Gln Lys Ala Ser Arg Glu Ser Pro Leu Gln Phe
    530                 535                 540

Ser Leu Asn Gly Gly Ser Glu Lys Gly Phe Gly Ile Phe Val Glu Gly
545                 550                 555                 560

Val Glu Pro Gly Ser Lys Ala Ala Asp Ser Gly Leu Lys Arg Gly Asp
                565                 570                 575

Gln Ile Met Glu Val Asn Gly Gln Asn Phe Glu Asn Ile Thr Phe Met
            580                 585                 590

Lys Ala Val Glu Ile Leu Arg Asn Asn Thr His Leu Ala Leu Thr Val
        595                 600                 605

Lys Thr Asn Ile Phe Val Phe Lys Glu Leu Leu Phe Arg Thr Glu Gln
    610                 615                 620

Glu Lys Ser Gly Val Pro His Ile Pro Lys Ile Ala Glu Lys Lys Ser
625                 630                 635                 640

Asn Arg His Ser Ile Gln His Val Pro Gly Asp Ile Glu Gln Thr Ser
                645                 650                 655

Gln Glu Lys Gly Ser Lys Lys Val Lys Ala Asn Thr Val Ser Gly Gly
            660                 665                 670

Arg Asn Lys Ile Arg Lys Ile Leu Asp Lys Thr Arg Phe Ser Ile Leu
        675                 680                 685

Pro Pro Lys Leu Phe Ser Asp Gly Gly Leu Ser Gln Ser Gln Asp Asp
    690                 695                 700

Ser Ile Val Gly Thr Arg His Cys Arg His Ser Leu Ala Ile Met Pro
705                 710                 715                 720

Ile Pro Gly Thr Leu Ser Ser Ser Ser Pro Asp Leu Leu Gln Pro Thr
                725                 730                 735

Thr Ser Met Leu Asp Phe Ser Asn Pro Ser Asp Ile Pro Asp Gln Val
            740                 745                 750

Ile Arg Val Phe Lys Val Asp Gln Gln Ser Cys Tyr Ile Ile Ile Ser
        755                 760                 765

Lys Asp Thr Thr Ala Lys Glu Val Val Phe His Ala Val His Glu Phe
    770                 775                 780

Gly Leu Thr Gly Ala Ser Asp Thr Tyr Ser Leu Cys Glu Val Ser Val
785                 790                 795                 800

Thr Pro Glu Gly Val Ile Lys Gln Arg Arg Leu Pro Asp Gln Phe Ser
                805                 810                 815

Lys Leu Ala Asp Arg Ile Gln Leu Asn Gly Arg Tyr Tyr Leu Lys Asn
            820                 825                 830

Asn Met Glu Thr Glu Thr Leu Cys Ser Asp Glu Asp Ala Gln Glu Leu
        835                 840                 845

Val Lys Glu Ser Gln Leu Ser Met Leu Gln Leu Ser Thr Ile Glu Val
    850                 855                 860

Ala Thr Gln Leu Ser Met Arg Asp Phe Asp Leu Phe Arg Asn Ile Glu
865                 870                 875                 880

Pro Thr Glu Tyr Ile Asp Asp Leu Phe Lys Leu Asn Ser Lys Thr Gly
                885                 890                 895

Asn Thr His Leu Lys Arg Phe Glu Asp Ile Val Asn Gln Glu Thr Phe
            900                 905                 910

Trp Val Ala Ser Glu Ile Leu Thr Glu Ala Asn Gln Leu Lys Arg Met
        915                 920                 925

Lys Ile Ile Lys His Phe Ile Lys Ile Ala Leu His Cys Arg Glu Cys
    930                 935                 940

Lys Asn Phe Asn Ser Met Phe Ala Ile Ile Ser Gly Leu Asn Leu Ala
945                 950                 955                 960

Ser Val Ala Arg Leu Arg Gly Thr Trp Glu Lys Leu Pro Ser Lys Tyr
                965                 970                 975

Glu Lys His Leu Gln Asp Leu Gln Asp Ile Phe Asp Pro Ser Arg Asn
            980                 985                 990

Met Ala Lys Tyr Arg Asn Ile Leu Ser Ser Gln Ser Met Gln Pro Pro
        995                 1000                1005

Ile Ile Pro Leu Phe Pro Val Val Lys Lys Asp Met Thr Phe Leu
    1010                1015                1020

His Glu Gly Asn Asp Ser Lys Val Asp Gly Leu Val Asn Phe Glu
    1025                1030                1035

Lys Leu Arg Met Ile Ser Lys Glu Ile Arg Gln Val Val Arg Met
    1040                1045                1050

Thr Ser Ala Asn Met Asp Pro Ala Met Met Phe Arg Gln Arg Ser
    1055                1060                1065

Leu Ser Gln Gly Ser Thr Asn Ser Asn Met Leu Asp Val Gln Gly
    1070                1075                1080

Gly Ala His Lys Lys Arg Ala Arg Arg Ser Ser Leu Leu Asn Ala
    1085                1090                1095

Lys Lys Leu Tyr Glu Asp Ala Gln Met Ala Arg Lys Val Lys Gln
    1100                1105                1110

Tyr Leu Ser Ser Leu Asp Val Glu Thr Asp Glu Glu Lys Phe Gln
    1115                1120                1125

Met Met Ser Leu Gln Trp Glu Pro Ala Tyr Gly Thr Leu Thr Lys
    1130                1135                1140

Asn Leu Ser Glu Lys Arg Ser Ala Lys Ser Ser Glu Met Ser Pro
    1145                1150                1155

Val Pro Met Arg Ser Ala Gly Gln Thr Thr Lys Ala His Leu His
    1160                1165                1170

Gln Pro His Arg Val Ser Gln Val Leu Gln Val Pro Ala Val Asn
    1175                1180                1185

Leu His Pro Ile Arg Lys Lys Gly Gln Thr Lys Asp Pro Ala Leu
    1190                1195                1200

Asn Thr Ser Leu Pro Gln Lys Val Leu Gly Thr Thr Glu Glu Ile
    1205                1210                1215

Ser Gly Lys Lys His Thr Glu Asp Thr Ile Ser Val Ala Ser Ser
    1220                1225                1230

Leu His Ser Ser Pro Pro Ala Ser Pro Gln Gly Ser Pro His Lys
    1235                1240                1245

Gly Tyr Thr Leu Ile Pro Ser Ala Lys Ser Asp Asn Leu Ser Asp
    1250                1255                1260

Ser Ser His Ser Glu Ile Ser Ser Arg Ser Ser Ile Val Ser Asn
    1265                1270                1275

Cys Ser Val Asp Ser Met Ser Ala Ala Leu Gln Asp Glu Arg Cys
    1280                1285                1290

Ser Ser Gln Ala Leu Ala Val Pro Glu Ser Thr Gly Ala Leu Glu
    1295                1300                1305

Lys Thr Glu His Ala Ser Gly Ile Gly Asp His Ser Gln His Gly
    1310                1315                1320

Pro Gly Trp Thr Leu Leu Lys Pro Ser Leu Ile Lys Cys Leu Ala
    1325                1330                1335

Val Ser Ser Ser Val Ser Asn Glu Glu Ile Ser Gln Glu His Ile
    1340                1345                1350

Ile Ile Glu Ala Ala Asp Ser Gly Arg Gly Ser Trp Thr Ser Cys
    1355                1360                1365

Ser Ser Ser Ser His Asp Asn Phe Gln Ser Leu Pro Asn Pro Lys
    1370                1375                1380

Ser Trp Asp Phe Leu Asn Ser Tyr Arg His Thr His Leu Asp Asp
    1385                1390                1395

Pro Ile Ala Glu Val Glu Pro Thr Asp Ser Glu Pro Tyr Ser Cys
    1400                1405                1410

Ser Lys Ser Cys Ser Arg Thr Cys Gly Gln Cys Lys Gly Ser Leu
    1415                1420                1425

Glu Arg Lys Ser Trp Thr Ser Ser Ser Ser Leu Ser Asp Thr Tyr
    1430                1435                1440

Glu Pro Asn Tyr Gly Thr Val Lys Arg Arg Val Leu Glu Ser Thr
    1445                1450                1455

Pro Ala Glu Ser Ser Glu Gly Leu Asp Pro Lys Asp Ala Thr Asp
    1460                1465                1470

Pro Val Tyr Lys Thr Val Thr Ser Ser Thr Glu Lys Gly Leu Ile
    1475                1480                1485

Val Tyr Cys Val Thr Ser Pro Lys Lys Asp Asp Arg Tyr Arg Glu
    1490                1495                1500

Pro Pro Pro Thr Pro Pro Gly Tyr Leu Gly Ile Ser Leu Ala Asp
    1505                1510                1515

Leu Lys Glu Gly Pro His Thr His Leu Lys Pro Pro Asp Tyr Ser
    1520                1525                1530

Val Ala Val Gln Arg Ser Lys Met Met His Asn Ser Leu Ser Arg
    1535                1540                1545

Leu Pro Pro Ala Ser Leu Ser Ser Asn Leu Val Ala Cys Val Pro
    1550                1555                1560

Ser Lys Ile Val Thr Gln Pro Gln Arg His Asn Leu Gln Pro Phe
    1565                1570                1575

His Pro Lys Leu Gly Asp Val Thr Asp Ala Asp Ser Glu Ala Asp
    1580                1585                1590

Glu Asn Glu Gln Val Ser Ala Val
    1595                1600

## Description

The invention relates to the integrity of cell-cell junctions in endothelial and epithelial cells and means and methods for increasing that integrity.

Integrity of the cell-cell junctions of epithelial and endothelial cells is important for maintaining homeostasis. Epithelial cell-cell adhesion regulates tissue morphogenesis as well as cell polarity [1], and is critical to prevent unwanted migration of cells such as metastasis. For example, the integrity of cell-cell junctions of endothelial cells prevents leakage of fluid and blood cells, including platelets into the surrounding tissue. Many pathological events induce vessel leakage or are caused by vessel leakage, for example but not limited to disorders such as sepsis, oedema, reperfusion injury and acute respiratory stress syndrome.

In the intestinal tract, the integrity and polarization of epithelial cell layers form a barrier against unwanted invasion of parasites in the body, bacteria and viruses, and against the unwanted transgression of compounds from the intestinal tract lumen to the rest of the body. A number of intercellular adhesion molecules are required to maintain polarization of epithelial cells. This organization is achieved by specialized junctions comprising adherens junctions (AJs), desmosomes, and tight junctions (TJs). AJs occur both in epithelial and endothelial cell-cell contacts and comprise, amongst others, cadherins, which interact with cadherins of neighbouring cells in a calcium-dependent manner to mediate cell-cell adhesion [2].

The Ras like small GTPases and their guanine nucleotide exchange factors (GEFs) mediate intercellular adhesion through the regulation of cytoskeletal organization, polarity and trafficking [3, 4]. Evidence suggests that, amongst others, activation of the small GTPase Rap is required to recruit E-cadherin at cell-cell contact sites [5]. Moreover, genetic and cell-based experiments indicate that Rap is required for proper AJs formation [6, 7]. However, the molecular mechanisms and the Rap GEFs involved in this process remain unclear. One of the Rap GEFs, PDZ-GEF2 (syn.:RapGEF6, RA-GEF2), a member of the PDZ-GEF family, is an exchange factor for Rap1 and Rap2 [8, 9], but its role in cell-cell junction integrity until now was unknown.

It is an object of the present invention to develop a screening method for selecting compounds that are useful for increasing cell-cell junction formation and maturation and/or useful in the treatment of syndromes related to blood vessel leakage and decreased epithelial cell wall integrity.

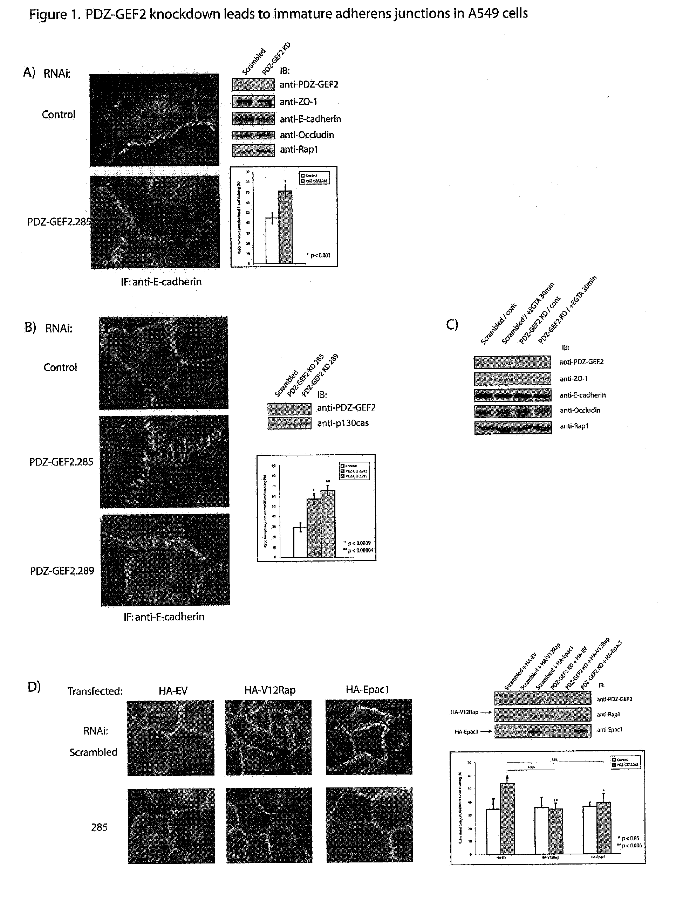

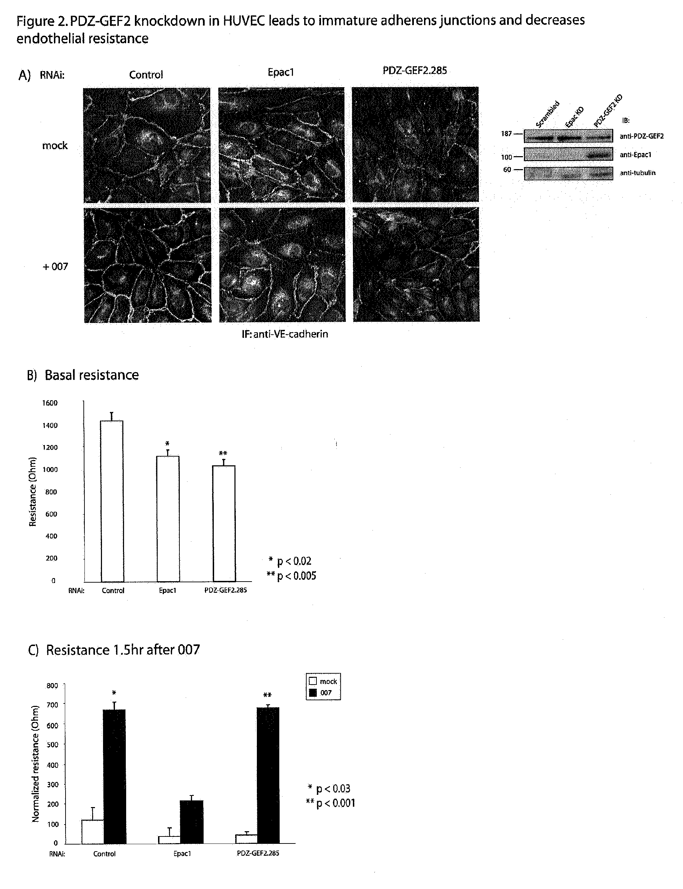

We discovered that PDZ-GEF2, a protein that was until now not considered to play a role in the integrity and/or in the maturation of cell-cell junctions, mediates the maturation of cell-cell junctions and improves barrier function. We have developed cells in which PDZ-GEF2 has been decreased by the action of siRNA. We discovered that a decrease or inactivation of PDZ-GEF2 leads to impaired or disrupted cell-cell contacts (Figures 1 and 2). By comparing these cells with cells with an expression of PDZ-GEF2 that is higher than that of the cells with decreased PDZ-GEF2, we can proceed to the selection of compounds that induce the activation of PDZ-GEF2 and inhibit leakage of fluids and cells through blood vessel walls and/or increase the integrity of epithelial cell-cell junctions. However, other methods can be used to identify compounds that activate PDZ-GEF2

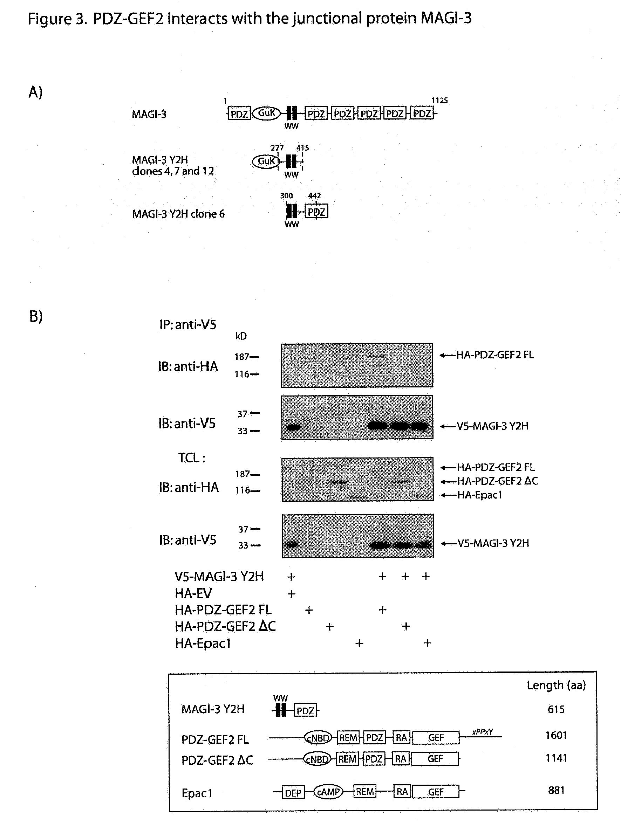

The present invention discloses that the Rap guanine nucleotide exchange factor PDZ-GEF2 mediates adherens junction maturation and cell-cell integrity. Using a yeast-two-hybrid screen, we show that PDZ-GEF2 associates with the junctional scaffold protein MAGI-3 (Figure 3). This is in contrast to Rap GEF PDZ-GEF1, which has been shown to associate with the junctional scaffolding molecules MAGI-1 and MAGI-2, and to bind and co-localize with the AJs protein β-catenin [10-14].

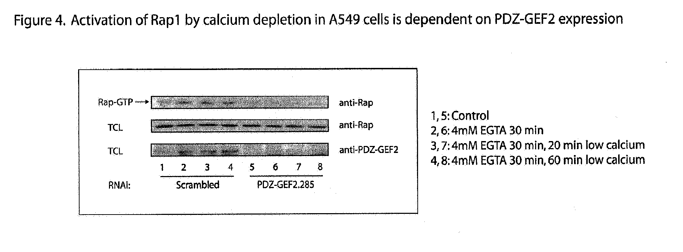

Furthermore, we disclose that decreasing the level of PDZ-GEF2 by PDZ-GEF2-knockdown mediated by siRNA in cells, for example in epithelial cells, abolishes Rap activation triggered by low calcium concentration (Figure 4). In addition, we disclose that PDZ-GEF2 knockdown leads to an immature adherens junction phenotype in cells, for example epithelial and endothelial cells. Using real-time measurement of electrical resistance across human umbilical vein cord cell monolayers, we show that PDZ-GEF2 depletion decreases transendothelial resistance, a measure for barrier function. This effect can be rescued with 8-pCPT-2'OMe-cAMP, an agonist of Epac, a GEF for Rap1 and Rap2, showing that activation of Rap 1 and/or Rap2 by PDZ-GEF2 is a critical event in the induction of barrier function. Since PDZ-GEF2 is a protein with similar function as Epac and also increases barrier function by activation of Rap1 and/or Rap2, this result implies that activation of PDZ-GEF2 by a selective compound will result in an increased barrier function of endothelial and epithelial cells.

Thus, the present invention discloses that increase and/or activation of PDZ-GEF2 increases the integrity of cell-cell junctions of cells, for example between endothelial and/or epithelial cells by mediating the activation of Rap.

This insight enables a new method to increase barrier function of endothelial and epithelial cells by compounds capable of increasing the activity of PDZ-GEF2. This invention now discloses a method, using a compound that activates PDZ-GEF2 to increase endothelial and epithelial barrier function. This compound is identified, for instance, but not exclusively, by screening for compounds that bind to PDZ-GEF2 *in vitro,* by screening for compounds that induce guanine nucleotide exchange activity of PDZ-GEF2 *in vitro,* or by screening for activation of PDZ-GEF2 in a cell. The present invention provides a method to increase the barrier function of a cell-cell junction, said method comprising the activation of a PDZ-GEF2 protein, wherein said PDZ-GEF2 protein comprises an amino acid sequence encoded by a gene corresponding to the RapGEF6 gene. Of course said PDZ-GEF2 protein also encompasses splice variants. Splice variants are sequences that occur naturally within the cells and tissues of individuals, but differ from the original sequence through alternative splicing of pre-mRNA.

This insight also enables new methods for selecting compounds capable of increasing endothelial and epithelial cell-cell integrity, thereby decreasing transport of fluid and cells through endothelial and/or epithelial barriers. For example, the present invention now discloses a method using an *in vitro* cell culture technique in which the status and integrity of cell-cell junctions can be measured, to select an activator or agonist for PDZ-GEF2. In one embodiment, two cell types are used, wherein in one cell type the formation and integrity of cellular junctions is at least in part inhibited by PDZ-GEF2 downregulation or knockdown (KD), for example mediated by siRNA (from here these cells are called PDZ-GEF2-KD cells). In the other cell type, PDZ-GEF2 is not or to a lesser extent down-regulated (from here these cells are called parental cells). Subsequently, a candidate compound of a plurality of compounds is administered to the cell cultures of the two cell types and the formation and/or maturation and/or integrity of cell-cell junctions are measured. Any difference in increase in the maturation or formation of the cell-cell junctions of parental cells compared to PDZ-GEF-KD cells, after contacting said cells with said compound indicates that said compound is capable of activating PDZ-GEF2 that is present in the cells. Said compound is then selected from said plurality of compounds as an agonist of PDZ-GEF2. Therefore, the present invention discloses a method for selecting an activator of PDZ-GEF2, said method comprising contacting a plurality of candidate compounds to at least two cells of a first cell type and at least two cells of a second cell type, both cell types capable of forming cell-cell junctions, measuring the maturation of cell-cell junctions before and after contacting said two cell types with said plurality of candidate compounds, and selecting a compound of said plurality of candidate compounds that is more capable of increasing the integrity of a cell-cell junction between said at least two cells of said first cell type, as compared to its capability of increasing the integrity of a cell-cell junction between cells of said second type, wherein in said second cell type the amount and/or activity of PDZ-GEF2 is decreased, compared to said first cell type.

Because the above-described method provides an activator of PDZ-GEF2, which increases the maturation of cell-cell junctions between cells, the invention discloses a method to increase barrier function of epithelial and endothelial cells by activation of PDZ-GEF by any method, including, but not exclusively, methods using chemical compounds and/or antibodies, and or peptides capable of activating PDZ-GEF2.

The present invention furthermore provides a cell wherein PDZ-GEF2 is at least partially downregulated, said cell characterized by immature cell-cell junctions and/or a decreased concentration of PDZ-GEF2 compared to a natural cell of the same kind. Said cell preferably comprises an epithelial cell. More preferably, said cell comprises an endothelial cell. Said cell is particularly suitable for testing compounds that activate PDZ-GEF2.

The present invention therefore provides the use of a cell according to the invention, preferably in combination with a parental cell, for the development of an assay to select an agonist of PDZ-GEF2 from a plurality of compounds and the use of a cell according to the invention for a selection assay for a compound improving the integrity of endothelial and/or epithelial cell-cell junctions.

In one preferred embodiment, a compound selected according to the method of the invention as described above does not necessarily bind directly to PDZ-GrEF2. For example, if an activating effect is achieved by binding of a compound selected by a method of the invention, to a substance that is capable of suppressing or preventing the activation of PDZ-GEF2, then, binding as described above results in at least partial decrease of the suppressing or activation-preventing effect of the substance and thereby in the activation of PDZ-GEF2. A compound selected according to a method of the invention and having such an effect is called hereinafter an indirect agonist. Therefore, the present invention now provides a method as described above, wherein said compound is an indirect agonist for PDZ-GEF2.

In another preferred embodiment, if activation of PDZ-GEF2 is achieved by direct binding of a compound as selected according to a method of the invention, then said compound, selected according to a method of the invention and having an activating effect by direct binding PDZ-GEF2, is called hereinafter a direct agonist. Therefore, the present invention now provides a method as described above, wherein a compound is selected which comprises a direct agonist of PDZ-GEF2. The effect of PDZ-GrEF2 on cell-cell junctions results in an increased integrity of cell-cell junctions, more particularly the integrity of adherens junctions. Therefore, the present invention now provides a method as described above, wherein said agonist is capable of increasing integrity of a cell-cell junction. Increased integrity of cell-cell junctions results in a decrease in permeability of an endothelial or epithelial cell wall or barrier for fluid and cells. Therefore, the present invention now provides a method as described above, wherein said agonist is capable of decreasing permeability of an endothelial and/or epithelial cell wall or barrier.

In one embodiment, a compound selected by a method according to the present invention as described above, binds preferably to PDZ-GEF2. Therefore, the present invention provides a method as described above, wherein said compound binds to PDZ-GEF2. In another preferred embodiment said compound also influences and/or binds to PDZ-GEF1 and in a preferred embodiment the capability of influencing or binding to both PDZ-GEF2 as PDZ-GEF1 enhances the effect of said compound on the integrity of endothelial and/or epithelial cell-cell junctions.

The invention as described above enables a skilled person to select a compound capable of binding to PDZ-GEF2 or a functional part or derivative thereof. Said compound preferably comprises a small compound or in another embodiment, a protein or proteinaceous compound. A functional part of a compound capable of binding to PDZ-GEF2 is defined as a part that has the same kind of biological properties in kind, not necessarily in amount. A functional derivative of a compound capable of binding to PDZ-GEF2 is defined as a compound which has been altered such that the biological properties of said compound are essentially the same in kind, not necessarily in amount.

In a preferred embodiment, the present invention herewith provides an agonist of PDZ-GEF2, said agonist selected by a method of the invention as described above. An agonist as selected according to the invention is very suitable for the treatment of disorders that are related to a decreased integrity of enclothe3ial and/or epithelial walls, such as for example but not limited to: vessel leakage, oedema, haemorrhages and diarrhoea. Therefore, the present invention provides the use of an agonist of PDZ-GEF2 according to the invention as described above as a medicament, and also the use of an agonist of PDZ-GEF2 according to the invention as described above for the manufacture of a medicament for decreasing vascular leakage and/or for increasing endothelial and/or epithelial integrity. Said medicament is preferably given by the oral route or by parenteral route for the treatment of disorders that are related to a decreased integrity of endothelial and/or epithelial walls, such as for example: vessel leakage, oedema, haemorrhages and diarrhoea. The medicament is preferably prepared by combining an agonist according to the present invention with a suitable carrier and/or diluent. Said carrier or diluent preferably comprises water, saline solution or another medium or carrier that is suitable and accepted for oral or parenteral administration. The present invention therefore discloses a pharmaceutical composition comprising an agonist of PDZ-GEF2 and a suitable carrier or diluent.
The invention is further explained by the examples, without being limited by them.

### Examples

### Materials and methods

### Cell culture

293T cells were maintained in DMEM (BioWhittaker) supplemented with 10% fetal bovine serum (FBS) (Cambrex), 1.2 mM L-Glutamine (BioWhittaker) and antibiotics (100 units/ml penicillin, 100µg/ml streptomycin) (BioWhittaker). A549 cells were cultured in RPMI (BioWhittaker) supplemented with 10% FBS and antibiotics as described above. HUVEC were isolated according to Jaffe et al. [16, 17] and cultured on 1% gelatin (Sigma) in EBM-2 Bulletkit culture medium (EBM-2 supplemented with EGM-2 SingleQuots (hEGF, hydrocortisone, fetal bovine serum, VEGF, hFGF-B, R3-IGF-1, ascorbic acid, GA-1000, heparin) (Clonetics). Second to fifth passage cells were used.

### Antibodies

Monoclonal antibodies 5D3 against Epac1 have been described previously [7]. Additional primary antibodies were purchased from AbCam (anti-E-Cadherin HecD1), GeneTex (anti-E-Cadherin HecD1), Invitrogen (anti-V5), Oncogene (anti-alpha-tubulin), Santa Cruz Biotechnology (anti-Rap), Sigma (anti-MAGI-3), Transduction Laboratories (p130cas, β-catenin, VE-cadherin: Cadherin-5), Zymed (anti-occludin, anti-ZO-1), anti-hemagglutinin (HA) monoclonal antibody 12CA5. Anti-mouse and anti-rabbit fluorescent secondary antibodies (FITC and TRITC) were from Molecular Probes.

### PDZ-GEF2 antibody production

To produce antibodies reactive against PDZ-GEF2, the first nucleotide binding domain of human PDZ-GEF2 corresponding to amino acids 1-123 was subcloned into the bacterial expression vector pGEX. Glutathione S-transferase (GST) fusion protein was produced and used to immunize rabbits.

### Yeast-two-hybrid screen

Full length PDZ-GEF2 was subcloned into the pB38 vector and used as a bait in a yeast-two-hybrid system with a human placenta cDNA library. The screen was performed by Hybrigenics as described in [18].

### Expression vectors and cloning/DNA construction

The following hemagglutinin (HA)-tagged expression vectors were described previously: pMT2-HA-PDZ-GEF2 full length and pMT2-HA-PDZ-GEF2 ΔC [9]; pMT2-HA-Epac1 [19]; pMT2-HA-V12Rap [20]. The mouse MAGI-3 cDNA clone IMAGp998A228567Q was obtained from the German Resource Center for Genome Research (RZPD) and used to clone the WW domains and the second PDZ domain of MAGI-3, which correspond to the interacting part found in the yeast-two-hybrid screen (clone 6). Amino acids 291-504 corresponding to the mouse WW-WW-PDZ domains of MAGI-3 were generated by PCR amplification and cloned into a Gateway entry vector (Invitrogen) using the following primer set: Forward 5'-GGGGACAAGTTTGTACAAAAAAGCAGGCTCTAGAGATGAGAATCTGGAACCG-3'; Reverse 5'-GGGGACCACTTTGTACAAGAAAGCTGGGTCTTAACGGCATA AAGTAAGGTTGACAT-3'. The MAGI-3 Y2H (clone 6) entry clone was recombined into a V5-pcDNA3 destination vector following the manufacturer's instructions. The V5-pcDNA3-MAGI-3 Y2H vector was sequenced to ensure proper identity of the amino acids.

### Transfection

293T and A549 cells were transfected using FuGENE6 reagent for 48 hr (Roche Diagnostics Corporation). For siRNA, A549 cells and HUVEC were transfected using oligofectamine (Invitrogen) for 24-48 hr according to the manufacturer's instructions. In the case of the HUVEC, transfection was repeated after 24 hr. Samples were analyzed 48 hr after the second round of transfection. Oligos sequences (Dharmacon): PDZ-GEF2 (285: GGATCCAACTTATATA GAA); (289: GACACGGATTGTATTA TTA); *Epac1 (GCACCTACGTCTGCAA CAA) and (CCATCATCCTGCGAGAAGA). 8-pCPT-2'0-Me-cAMP/8-pCPT-2'-OMe-cAMP (Biolog) was used at a final concentration of 100 µM. Quantification of the PDZ-GEF2 knockdown was performed using Scion Image (Scion Corporation).

### Immunofluorescence

A549 cells transfected with DNA expression vectors and/or siRNA oligos were plated on coverslips. 24 or 48 hr later, cells were rinsed twice with ice cold PBS and fixed in a solution of 3.8% formaldehyde, permeabilized in 0.1% Triton X-100 and blocked with 2% BSA in PBS at room temperature for 1h or overnight at 4°C. The coverslips were incubated with the indicated primary antibodies for 1h followed by 30min incubation with the Alexa secondary antibodies, then mounted on glass slides and dried overnight. The cells were examined using a Zeiss Axiokop2 microscope or by conforcal microscopy. Pictures were taken at 40x magnification and were arranged and resized using Adobe Photoshop CS. The values are presented as the average of the number of junctions scoring immature over the total E-cadherin junction staining ± standard deviation of the mean (SE).

### Calcium switch experiments

A549 cells transfected with scrambled or PDZ-GEF2.285 oligo's were replated in growth media 24hr before calcium switch. The dishes were then rinsed twice with PBS and serum-free and calcium-free media containing 4mM EGTA was added to the cells for 30 minutes at 37°C to disrupt E-cadherin-mediated cell-cell contacts. Following calcium chelation, cells were lysed or incubated in media supplemented with low calcium (20µM) for 20 and 60 minutes.

### Immunoprecipitation and western blotting

Cells were washed in ice-cold PBS and lysed in modified RIPA buffer (150 mM NaCl, 50 mM Tris-HCl pH 7.4, 1% Nonidet P-40) containing protease inhibitors, sodium vanadate and sodium fluoride. Cell lysates were cleared by centrifugation and rotated with anti-V5 or anti-HA antibodies and protein A-Agarose at 4°C for 2 h. Precipitates were washed four times in lysis buffer, resuspended in SDS sample buffer, resolved by 8% SDS-PAGE and transferred to polyvinylidene difluoride membrane (PVDF; Immobilon, Millipore). The membranes were blocked in 1% bovine serum albumin (BSA) and 1% non-fat milk, followed by incubation with primary antibodies overnight at 4 °C. Horseradish peroxidase-conjugated anti-mouse or anti-rabbit was used as a secondary antibody and the blot was developed using enhanced chemiluminescence.

### Endothelial permeability assay

To assess endothelial permeability, we measured electrical resistance continuously across endothelial cell monolayer using the methodology known as "Electric Cell-Substrate Impedance Sensing" (ECIS) [21]. Briefly, HUVEC were grown to confluence on small gold electrodes and ECIS was subsequently monitored for up to 15 hr. Data was analyzed using the Applied Biophysics software.

### Results

### The impact of PDZ-GEF2 knockdown on junction formation

In epithelial cells, the presence of E-cadherin protein mediates the establishment of cell-cell contacts (reviewed in [22]), The presence of E-cadherin in mature cell-cell contacts can be visualized *in vitro*. Therefore, PDZ-GEF2 depleted A549 cells were replated on glass coverslips and E-cadherin staining was performed. Treatment with PDZ-GEF2 siRNAi for 48 hr effectively reduced the levels of endogenous PDZ-GEF2 protein to 64% and 52% of baseline (Figure 1A and B, oligo 285 and 289 respectively). Remarkably, PDZ-GEF2 depletion was associated with suppression of cell-cell contacts maturation 24 and 48 hr following seeding of the cells (Figures 1A and B respectively). The junctions depicted are zipper-like structures, as seen in epithelial cells establishing initial cell-cell contacts [23]. We quantified the amount of these immature junctions and obtained approximately twice as much in the absence of PDZ-GEF2 (graphs Figure 1A and B). A similar pattern of localization was observed for β-catenin, another marker of adherens junction integrity (data not shown). To exclude impairment of E-cadherin or other junctional proteins expression in absence of PDZ-GEF2, we examined the levels of E-cadherin, ZO-1 and occludin in cells treated or not treated with 4mM EGTA for 30 minutes and found equal amounts in scrambled and PDZ-GEF2 siRNA treated cells (Figure 1A and C). Interestingly, the PDZ-GEF2 knockdown phenotype (PDZ-GEF2-KD) is similar to the one observed in Rap1A depleted cells (data not shown), providing evidence that PDZ-GEF2 is required for Rap activation and further supporting a role for Rap in junction establishment.

### Expression of activated Rap or Epac1 rescues adherens junction maturation in PDZ-GEF2 depleted cells (PDZ-GEF2-KD)

To confirm that the immature junction phenotype was caused by PDZ-GEF2 depletion that downregulated Rap activation, activated Rap (V12Rap) and Epac1 were reexpressed in scrambled or PDZ-GEF2 siRNA transfected cells and anti-E-cadherin staining was performed. Expression of these constructs is known to lead to Rap activation and significantly restored junction maturation to levels observed in cells transfected with the scrambled oligo and empty vector (Figure 1D). We concluded that Rap activation by PDZ-GEF2 is critical for cell-cell contact maturation in A549 cells.

### Increased endothelial permeability in HUVEC depleted in PDZ-GEF2 (PDZ-GEF2-KD)

We next showed the role of PDZ-GEF2 for adherens junction formation in endothelial cells. HUVEC were transfected as described and seeded on glass coverslips or on electrodes for ECIS measurement of cellular resistance. Experiments were performed when cells formed a confluent layer. In Figure 2A, VE-cadherin staining of PDZ-GEF2 depleted HUVEC (PDZ-GEF2-KD) clearly showed zipper-like, immature junctions, whereas scrambled siRNA had no effect on junction appearance. It is known that immature junctions correlate with increased cell permeability and that the Rap GEF Epac1 regulates this process [24-27]. Therefore, we studied electrical resistance of HUVEC treated with Epac1 and PDZ-GEF2 siRNA. As previously reported, basal electrical resistance was decreased in Epac1 depleted cells (Figure 2B). The morphological phenotype was confirmed by electrical resistance functional assay. As expected, the reduced maturation of cell-cell contacts in absence of PDZ-GEF2 correlated with a 30% decrease in basal electrical resistance (Figure 2B). Activation of Rap through Epac1 by its specific agonist 8-pCPT-2'-OMe-cAMP is known to decrease cell permeability of endothelial cells by tightening the junctions [26]. Accordingly, a 30 minutes stimulation with 8-pCPT-2'-OMe-cAMP (100µM) increased resistance in scrambled siRNA cells and the decreased resistance observed in PDZ-GEF2 depleted cells was completely rescued by 8-pCPT-2'-OMe-cAMP (Figure 2C). As a control, HUVEC transfected with Epac1 siRNA were stimulated with 8-pCPT-2'-OMe-cAMP and a minimal effect on electrical resistance was observed. These findings indicate that PDZ-GEF2 is required to maintain endothelial cell integrity. Moreover, the impaired junction maturation was consistent with decreased electrical resistance in PDZ-GEF2 depleted HUVEC. Furthermore, activation of Epac by 8-pCPT-2'-OMe-cAMP could rescue the defect

### Identification of MAGI-3 as a PDZ-GEF2-interacting protein.

In a yeast two hybrid screen using PDZ-GEF-2 as a bait, we found MAGI-3 as an interaction partner of PDZ-GEF-2. Said MAGI-3 protein comprises a PDZ (PSD-95/disc large/zona occludens 1) domain and a GuK (guanylate kinase) domain and is a member of the MAGI family. Furthermore HA-PDZ-GEF2 full length co-immunoprecipitates with V5-MAGI-3 clone 6 when co-expressed in 293T cells (Figure 3)

### PDZ-GEF-2 required for Rap1 activation

To investigate whether PDZ-GrEF 2 is required for Rap activation during junction remodelling, we introduced PDZ-GEF2 siRNA in A549 cells and determined its effect on Rap-GTP levels upon disruption of cell junctions by EGTA. In control siRNA treated cells, EGTA induced Rap1 activation (Fig. 4A, lane 2), compatible with previous results. To exclude effects of EGTA, we have replaced the EDTA for low Ca²⁺ (20µM). Under these conditions junctions are not formed (data not shown) and Rap 1 remains elevated (Fig. 4A, lanes 3 and 4). In PDZ-GEF2 depleted cells, EGTA treatment was ineffective in increasing Rap1-GTP levels (Fig. 4A, lanes 6-8), showing that PDZ-GEF2 is required for Rap activation triggered by the disruption of E-cadherin-based junctions.

### Legends to the figures

### Figure 1. PDZ-GEF2 depletion in epithelial cells impairs adherens junction formation.

A and B, PDZ-GEF2 knockdown leads to immature AJs. A549 cells transfected with scrambled and PDZ-GEF2 siRNAs were replated on glass coverslips for 24 hr (A) and 48 hr (B) following which the cells were fixed and immunostained with anti-E-cadherin (HecD1) antibody. In both panels, cells depleted in PDZ-GEF2 using the siRNA 1 (285) or 2 (289) show immature cell-cell contacts. E-cadherin junction staining was quantified by analyzing at least 350 cells from 10 different fields and scoring the number of cells that exhibited mature or immature junctions according to the ratio of pixel intensity / area of the cell-cell junction. The values are presented as the average of the number of junctions scoring immature over the total E-cadherin junction staining ± standard deviation of the mean (SE). Quantification of the PDZ-GEF2 knockdown was performed using Scion Image and normalized with the corresponding loading control. C, Expression levels of junctional proteins ZO-1, E-cadherin, Occludin are not affected by the absence of PDZ-GEF2. A549 cells transfected with scrambled and PDZ-GEF2 siRNAs were replated and were left untreated or treated with 4mM EGTA for 30 minutes and equal amount of proteins were analyzed by SDS-PAGE and immunoblot. D, Activated Rap1 and Epac1 rescue adherens junctions maturation in absence of PDZ-GEF2. A549 cells were treated with scrambled and PDZ-GEF2 siRNAs overnight and transfected with HA-tagged constructs (empty vector, V12Rap or Epac1) the next day. The cells were replated on glass coverslips and fixed and immunostained 24 hr later. E-cadherin junction staining was quantified and described in A and B. Averages presented in the graph are means of three independent experiments. * p < 0.05, ** p < 0.006. (S₋V vs 285_EV: p<0.05; 285_EV vs 285_V12Rap: p<0.006; 285_EVvs 285_Epacl: p<0.05). t-test A, * p<fl.003; B, * p < 0.0009, ** p<0.00004.

### Figure 2. PDZ-GEF2 knockdown in HUVECs leads to immature adherens junction and decreases endothelial resistance.

A, PDZ-GEF2 knockdown leads to immature VE-cadherin-based junctions in endothelial cells. HUVEC were transfected twice with scrambled, Epac1 or PDZ-GEFs siRNA and replated on glass coverslips. The cells were fixed when they formed a monolayer and immunostained with anti-VE-cadherin antibody. B, Basal electrical resistance across HUVEC monolayers. Cells were treated as described in A and replated on electrodes. ECIS was measured continuously for 15hr and the values reported are the final time points. C, 007 increases endothelial resistance in HUVEC and rescues the PDZ-GEF2 knockdown phenotype. The values reported correspond to the endothelial resistance measured 90min after adding 007 to the culture media. * p < 0.03 control mock vs 007; * p < 0.001 PDZ-GEF2.285 mock vs 007.

### Figure 3. Identification of MAGI-3 as a PDZ-GEF2-interacting protein.

A, Schematic representation of the full length MAGI-3 protein and the MAGI-3 clones 4, 7, 12 and 6 identified in the yeast-two hybrid screen of human placenta cDNA library. PDZ, PSD-95/disc large/zona occludens 1 domain; GuK, guanylate kinase domain. B, Co-immunoprecipitation of HA-PDZ-GEF2 full length with V5-MAGI-3 clone 6 from 293T cells (top panel). 293T cells transfected with 3 µg of plasmid cDNAs encoding V5-MAGI-3 clone 6, HA-empty vector, HA-PDZ-GEF2 full length, HA-PDZ-GEF2 C-terminal truncated and HA-Epac1 and lysed in modified RIPA 48h post transfection. V5-MAGI-3 clone 6 was immunoprecipitated using V5 antibody and protein A beads. Samples were subjected to 10% SDS-PAGE electrophoresis, followed by immunoblotting using anti-V5 and anti-HA (12CA5) antibodies. Neither HA-PDZ-GEF2 C-terminal truncated nor HA-Epac1 coimmunoprecipitate with V5-MAGI-3 clone 6 (WW-WW-PDZ). Expression of the constructs is shown on the bottom panels (TCL). The results are representative of three independent experiments.

### Figure 4. Activation of Rap1 by calcium depletion in A549 cells is dependent on PDZ-GEF2 expression.

A549 cells were transfected with scrambled or PDZ-GEF2 RNAi oligos and replated 24 hr before the calcium switch at a density ensuring confluency at the time of the experiment. Cells were rinsed with PBS and treated with 4mM EGTA for 30 minutes to disrupt Ca²⁺-dependent cell adhesion, following which the cells were lysed or rinsed with PBS and incubated in serum-free media containing 20µM calcium. The cells were lysed at the indicated time points and the samples were incubated with immobilized GST-RalGDS-Ras binding domain to precipitate active (GTP-bound) Rap.

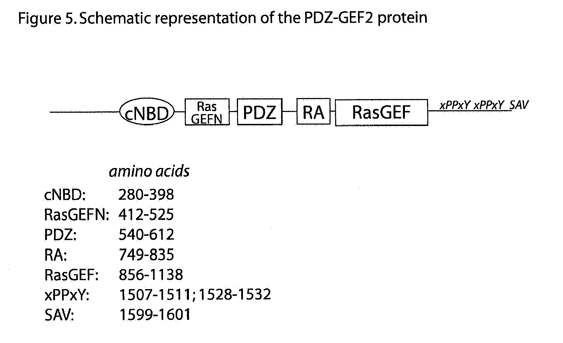

### Figure 5. Schematic representation of the PDZ-GEF2 protein.

PDZ-GEF2 domain organization: cNBD, cyclic nucleotide binding domains; RasGEFN, N-terminal Ras-GEF domain; PDZ, domain present in PSD-95, Dgl, and ZO-1; RA, Ras-associating domain; RasGEF, guanine-nucleotide dissociation stimulator CDC25 homology domain; xPPxY, protein-protein interaction PY-motifs; SAV, C-terminal PDZ binding motif.

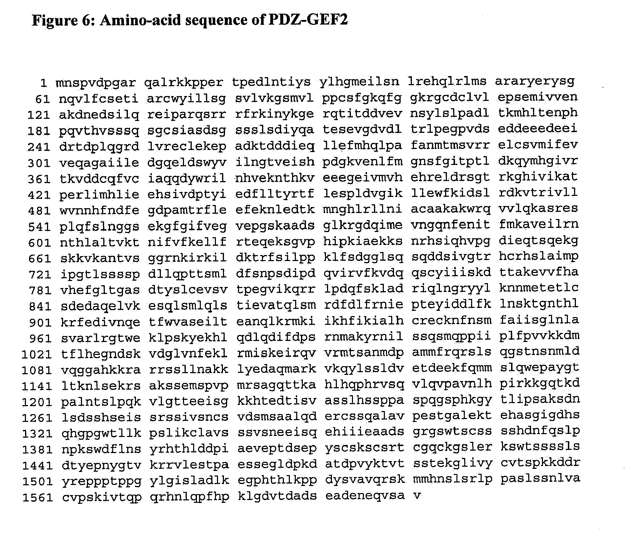

### Figure 6: Amino-acid sequence of PDZ-GEF2.

Indicated is the amino acid sequence of PDZ-GEF2, a protein encoded by the RapGEF6 gene. The gene may encode splice variants of PDZ-GEF2, proteins which are included in the present invention.

### References

1. Chen, X. and B.M. Gumbiner, Crosstalk between different adhesion molecules. Curr Opin Cell Biol, 2006. 18(5): p. 572-8.
2. Vasioukhin, V. and E. Fuchs, Actin dynamics and cell-cell adhesion in epithelia. Curr Opin Cell Biol, 2001. 13(1): p. 76-84.
3. Bos, J.L., Linking Rap to cell adhesion. Curr Opin Cell Biol, 2005. 17(2): p. 123-8.
4. Braga, V.M. and A.S. Yap, The challenges of abundance: epithelial junctions and small GTPase signalling. Curr Opin Cell Biol, 2005. 17(5): p. 466-74.
5. Hogan, C., et al., Rap1 regulates the formation of E-cadherin-based cell-cell contacts. Mol Cell Biol, 2004. 24(15): p. 6690-700.
6. Knox, A.L. and N.H. Brown, Rap1 GTPase regulation of adherens junction positioning and cell adhesion. Science, 2002. 295(5558): p. 1285-8.
7. Price, L.S., et al., Rap1 regulates E-cadherin-mediated cell-cell adhesion. J Biol Chem, 2004. 279(34): p. 35127-32.
8. Gao, X., et al., Identification and characterization of RA-GEF-2, a Rap guanine nucleotide exchange factor that serves as a downstream target of M-Ras. J Biol Chem, 2001. 276(45): p. 42219-25.
9. Kuiperij, H.B., et al., Characterisation of PDZ-GEFs, a family of guanine nucleotide exchange factors specific for Rap1 and Rap2. Biochim Biophys Acta, 2003. 1593(2-3): p. 141-9.
10. Dobrosotskaya, LY. and G.L. James, MAGI-1 interacts with beta-catenin and is associated with cell-cell adhesion structures. Biochem Biophys Res Commun, 2000. 270(3): p. 903-9.
11. Kawajiri, A., et al., Identification of a novel beta-catenin-interacting protein. Biochem Biophys Res Commun, 2000. 273(2): p. 712-7.
12. Mino, A., et al., Membrane-associated guanylate kinase with inverted orientation (MAGI)-1 /brain angiogenesis inhibitor 1-associated protein (BAP1) as a scaffolding molecule for Rap small G protein GDP/GTP exchange protein at tight junctions. Genes Cells, 2000. 5(12): p. 1009-16.
13. Ohtsuka, T., et al., nRap GER: a novel neural GDP/GTP exchange protein for rap1 small G protein that interacts with synaptic scaffolding molecule (S-SCAM). Biochem Biophys Res Commun, 1999. 265(1): p. 38-44.
14. Sakurai, A., et al., MAGI-1 is required for Rap1 activation upon cell-cell contact and for enhancement of vascular endothelial cadherin-mediated cell adhesion. Mol Biol Cell, 2006. 17(2): p. 966-76.
15. Arndt, K. and K. Muller, Protein engineering protocols Methods in molecular biology. Vol. 352. 2007. 95-109.
16. Jaffe, E.A., et al., Culture of human endothelial cells derived from umbilical veins. Identification by morphologic and immunologic criteria. J Clin Invest, 1973. 52(11): p. 2745-56.
17. Willems, C., et al., Media conditioned by cultured human vascular endothelial cells inhibit the growth of vascular smooth muscle cells. Exp Cell Res, 1982. 139(1): p. 191-7.
18. Rain, J.C., et al., The protein-protein interaction map of Helicobacter pylori. Nature, 2001. 409(6817): p. 211-5.
19. de Rooij, J., et al., PDZ-GEF1, a guanine nucleotide exchange factor specific for Rap1 and Rap2. J Biol Chem, 1999. 274(53): p. 38125-30.
20. Zwartkruis, F.J., et al., Extracellular signal-regulated activation of Rap1 fails to interfere in Ras effector signalling. Embo J, 1998. 17(20): p. 5905-12.
21. Tiruppathi, C., et al., Electrical method for detection of endothelial cell shape change in real time: assessment of endothelial barrier function. Proc Natl Acad Sci USA, 1992. 89(17): p. 7919-23.
22. Lien, W.H., O. Klezovitch, and V. Vasioukhin, Cadherin-catenin proteins in vertebrate development. Curr Opin Cell Biol, 2006. 18(5): p. 499-506.
23. Vasioukhin, V., et al., Directed actin polymerization is the driving force for epithelial cell-cell adhesion. Cell, 2000. 100(2): p. 209-19.
24. Cullere, X., et al., Regulation of vascular endothelial barrier function by Epac, a cAMP-activated exchange factor for Rap GTPase. Blood, 2005. 105(5): p. 1950-5.
25. Fukuhara, S., et al., Cyclic AMP potentiates vascular endothelial cadherin-mediated cell-cell contact to enhance endothelial barrier function through an Epac-Rap1 signaling pathway. Mol Cell Biol, 2005. 25(1): p. 136-46.
26. Kooistra, M.R., et al., Epac1 regulates integrity of endothelial cell junctions through VE-cadherin. FEBS Lett, 2005. 579(22): p. 4966-72.
27. Wittchen, E.S., et al., Rap1 GTPase inhibits leukocyte transmigration by promoting endothelial barrier function. J Biol Chem, 2005. 280(12): p. 11675-82.

## Claims

1. A method to increase the barrier function of a cell-cell junction, said method comprising activating a PDZ-GEF2 protein.

2. A method according to claim 1, wherein said method comprises the use of an indirect agonist of PDZ-GEF2.

3. A method according to claim 1, wherein said method comprises the use of a direct agonist of PDZ-GEF2.

4. A method according to claim 2 or 3, wherein said agonist increases integrity of a cell-cell junction.

5. A method according to any of claims 2 to 4, wherein said agonist decreases permeability of an endothelial and/or epithelial cell layer.

6. A method according to any one of claims 1 to 5, wherein said agonist binds to PDZ-GEF2.

7. A method for selecting an activator of PDZ-GEF2, said method comprising contacting a plurality of candidate compounds with at least two cells of a first cell type and at least two cells of a second cell type, both cell types capable of forming cell-cell junctions, measuring the maturation of cell-cell junctions before and after contacting said two cell types with said plurality of candidate compounds, and selecting a compound of said plurality of candidate compounds that is more capable of increasing the integrity of a cell-cell junction between said at least two cells of said first cell type, as compared to its capability of increasing the integrity of a cell-cell junction between cells of said second type, wherein in said second cell type the amount and/or activity of PDZ-GEF2 is decreased, compared to said first cell type.

8. A method according to claim 7, wherein said compound comprises an indirect agonist of PDZ-GEF2.

9. A method according to claim 7, wherein said compound comprises a direct agonist of PDZ-GEF2.

10. A method according to any of claims 7 to 9, wherein said agonist increases integrity of cell-cell junction.

11. A method according to any of claims 7 to 10, wherein said agonist decreases permeability of an endothelial and/or epithelial wall.

12. A method according to any one of claims 7 to 11, wherein said compound binds to PDZ-GEF2.

13. A method to select a compound capable of binding to PDZ-GEF2, said method comprising producing protein and/or fragments and/or peptides of 5 to 100 amino acids of a protein having an amino acid sequence corresponding to amino acid sequence 1-1601 of PDZ-GEF2, contacting a plurality of candidate compounds to said protein and/or fragments and/or peptides of 5 to 100 amino acids of a protein having an amino acid sequence corresponding to amino acid sequence 1-1601 of PDZ-GEF2, and selecting a compound that binds to said protein, and/or one or more fragments and/or one or more peptides, contacting said compound with said PDZ-GEF2, and determining whether said compound is capable of binding to PDZ-GEF2.

14. An agonist of PDZ-GEF2, said agonist selected by a method according to any of claims 7 to 13.

15. Use of an agonist of PDZ-GEF2 according to claim 14 as a medicament.

16. Use of an agonist of PDZ-GEF2 according to claim 14 for the manufacture of a medicament for decreasing vascular leakage and/or for increasing epithelial integrity.

17. A pharmaceutical composition comprising an agonist of PDZ-GEF2 according to claim 14 and a suitable carrier.

18. A cell wherein PDZ-GEF2 is at least partially downregulated, said cell **characterized by** immature cell-cell junctions and/or a decreased concentration of PDZ-GEF2 compared to a natural cell of the same kind.

19. Use of a cell according to claim 18 for a selection assay for an agonist of PDZ-GEF2.

20. Use of a cell according to claim 18 for a selection assay for a compound improving the integrity of endothelial and/or epithelial cell-cell junctions
